Europäisches Patentamt

European Patent Office

Office européen des brevets

(19)

(11) Numéro de publication: **0 237 402 B1**

(12)

# FASCICULE DE BREVET EUROPEEN

(45) Date de publication de fascicule du brevet: **01.04.92**

(51) Int. Cl.5: **C01B 15/022**, C07C 1/20, C07C 11/09

(21) Numéro de dépôt: **87400424.5**

(22) Date de dépôt: **26.02.87**

(54) **Procédé de fabrication conjointe de peroxyde d'hydrogène et d'isobutène.**

(30) Priorité: **14.03.86 FR 8603674**

(43) Date de publication de la demande:
**16.09.87 Bulletin 87/38**

(45) Mention de la délivrance du brevet:
**01.04.92 Bulletin 92/14**

(84) Etats contractants désignés:
**AT BE CH DE ES FR GB GR IT LI LU NL SE**

(56) Documents cités:
**US-A- 3 737 518**
**US-A- 3 891 748**
**US-A- 4 547 354**

(73) Titulaire: **ATOCHEM**
**4 & 8, Cours Michelet La Défense 10**
**F-92800 Puteaux(FR)**

Titulaire: **OXYSYNTHESE**
**6, rue Cognacq-Jay**
**F-75007 Paris(FR)**

(72) Inventeur: **Schirmann, Jean-Pierre**
**49, Chemin de la Glacière**
**F-69600 Oullins(FR)**
Inventeur: **Pralus, Michèle**
**3, rue des Grasses**
**F-69450 Saint-Cyr au Mont-d'Or(FR)**

(74) Mandataire: **Vesin, Jacques et al**
**L'AIR LIQUIDE, SOCIETE ANONYME POUR**
**L'ETUDE ET L'EXPLOITATION DES PROCE-**
**DES GEORGES CLAUDE 75, quai d'Orsay**
**F-75321 Paris Cédex 07(FR)**

## Description

La présente invention concerne la fabrication conjointe de peroxyde d'hydrogène et d'isobutène à partir d'hydroperoxyde de tertiobutyle.

Elle concerne plus particulièrement une telle préparation réalisée en présence d'un composé à caractère acide.

Depuis plusieurs décennies, la description de divers procédés montre que l'hydroperoxyde de tertiobutyle, désigné dans ce qui suit par TBHP, ne conduit pas dans ces conditions à la formation conjointe de peroxyde d'hydrogène et d'isobutène, et tend donc à infirmer les dires de John E. LEFFLER rapportés dans Chem Rev 45, p. 385-417, 1949, affirmant une telle possibilité, en l'absence de tout support expérimental.

Ainsi, selon les brevets des Etats-Unis d'Amérique n° 2.522015 et 2.522016, il y a production de di-organo peroxyde sans formation de peroxyde d'hydrogène.

Selon le brevet US n° 3.917.709 est obtenu par contre, un mélange d'acétone et de méthanol.

De plus, selon le brevet US n° 3.737.518, on obtient une mole de tertiobutanol et une mole de peroxyde d'hydrogène à partir d'une mole d'hydroperoxyde de tertiobutyle et d'une mole d'eau, en présence par exemple, comme composé à caractère acide fort, d'une résine échangeuse d'ions sous forme acide.

Enfin, le brevet US 4.547.354 a pour objet l'obtention de peroxyde d'hydrogène par hydrolyse d'hydroperoxyde de tertiobutyle catalysée par de l'acide sulfurique, en présence de solvant organique pour faciliter la séparation des phases organique et aqueuse contenant respectivement du tertiobutanol et des sous-produits d'hydrolyse : peroxyde de ditertiobutyle, acétone et méthanol, et du peroxyde d'hydrogène.

Or, il a été trouvé selon l'invention un procédé qui atteint ce but en soumettant l'hydroperoxyde de tertiobutyle en milieu aqueux à l'action d'une résine échangeuse d'ions sous forme acide, la dite résine étant choisie parmi les résines échangeuses de type gel à acidité forte, stables en milieu aqueux à la température de celui-ci, comprise entre 60 et 150°C. En particulier, le maintien du milieu aqueux à une température comprise entre 80 et 120°C, permet d'envisager d'obtention de résultats industriels intéressants.

Il est le plus généralement nécessaire d'engager une quantité de résine, précédemment définie, correspondant à au moins 0,005 équivalent H$^+$ par mole d'hydroperoxyde de tertiobutyle mis en oeuvre.

Les résines dont la matrice est constituée par des chaines polystyréniques pontées avec du divinylbenzène et dont les groupements acides sont des groupements -SO$_3$H conviennent bien à la réalisation du procédé selon l'invention.

Les résines qui présentent dans le domaine des rayons de pores inférieurs à 0,1 $\mu$m, un volume poreux inférieur à 0,1 cm3/g et, dans le domaine des rayons de pores supérieurs à 0,1 $\mu$m, un volume poreux supérieur à 0,3 cm3/g, sont avantageuses dans la conduite du procédé.

La durée de l'opération, qui peut être réalisée à une presssion égale ou supérieure à la pression atmosphérique, en réacteur agité ou par passage de l'hydroxyde de tertiobutyle en milieu aqueux, à travers un lit fixe de résine échangeuse de type gel à acidité forte, peut varier dans d'assez larges limites, généralement comprises entre 1 heure et 6 heures, et le plus souvent comprise entre 3 et 5 heures.

Les exemples suivants, donnés à titre indicatif mais non limitatif, illustrent l'invention.

Dans chacun de ces exemples, la caractérisation et l'évaluation quantitative du peroxyde d'hydrogène formé conjointement à l'isobutène ont été assurées grâce à des méthodes telles que la Résonance Magnétique Nucléaires RMN$^{17}$O et la réduction de Ce$^{4+}$.

### EXEMPLE 1

Dans un réacteur en verre de 250 cm3, équipé d'un réfrigérant et muni d'une agitation magnétique, on introduit 30 g de résine gel, vendue sous la marque commerciale DOWEX 50W-X8® humide (100-200 Mesh), forme acide, par la Sociéte DOW CHEMICAL, avec un taux d'humidité de 53 %, et 180 g de solution aqueuse d'hydroperoxyde de tertiobutyle à 10 % en poids. Après cinq heures à 90°C, sous agitation, on dose 0,072 mole de peroxyde d'hydrogène, ce qui correspond à un rendement de 36 % en H$_2$O$_2$. On recueille 3,6 g d'isobutène (0,064 mole) par piégeage après le réfrigérant, branché sur l'évant du réacteur. Le rendement en isobutène est de 32 %.

### EXEMPLE 2

Dans l'appareillage de l'exemple 1, 145 g de solution aqueuse d'hydroperoxyde de tertiobutyle à 70 % en poids, 35 g d'eau et 30 g de résine gel DOWEX 50W-X8® humide (100-200 Mesh) forme H$^+$, sont agités pendant 3 heures 30 mn à 90°C. Le rendement en H$_2$O$_2$ est de 4,1 % et le rendement en isobutène de 5,2 %.

### EXEMPLE 3

On reprend les conditions de l'exemple 2, en remplaçant la résine DOWEX 50W-X8® par la résine gel Orzelith RS-120 humide, sous forme acide, commercialisée par la Société Permutit humide sous forme sodium. Après quatres heures sous agitation à 90°C, le rendement en $H_2O_2$ est de 3,5 % et le rendement en isobutène est de 3,2 %.

EXEMPLE 4

On reprend les conditions de l'exemple 1, en remplaçant la résine DOWEX 50W-X8® par la résine gel Amberlite IR-120 humide forme acide, commercialisée par la Société Rohm et Haas avec un taux d'humidité de 44-48 %. Le rendement en $H_2O_2$ est de 31,5 % et le rendement en isobutène est de 13,5 %.

EXEMPLE 5

Dans l'appareillage de l'exemple 1, 30 g de résine DOWEX 50W-X8® (100-200 Mesh) humide, sous forme acide, 112 g d'une solution aqueuse d'hydroperoxyde de tertiobutyle à 70 % en poids et 50 g d'eau, sont agités pendant cinq heures à 70°C. Le rendement en peroxyde d'hydrogène est de 2,1 % et le rendement en isobutène de 0,5 %.

**Revendications**

1. Procédé de production conjointe de peroxyde d'hydrogène et d'isobutène à partir d'hydroperoxyde de tertiobutyle dans lequel l'hydroperoxyde de tertiobutyle est soumis en milieu aqueux à une température comprise entre 60°C et 150°C à l'action d'une résine échangeuse d'ions sous forme acide, caractérisé en ce que la résine échangeuse d'ions est une résine de type gel à acidité forte stable dans le milieu aqueux.

2. Procédé de fabrication conjointe de peroxyde d'hydrogène et d'isobutène selon la revendication 1, caractérisé en ce que la température du milieu aqueux est comprise entre 80 et 120°C.

3. Procédé de fabrication conjointe de peroxyde d'hydrogène et d'isobutène selon l'une des revendications 1 et 2, caractérisé en ce que la quantité de résine échangeuse d'ions de type gel à acidité forte engagée correspond au moins à 0,005 équivalent $H^+$ par mole d'hydroperoxyde de tertiobutyle mis en oeuvre.

4. Procédé de fabrication conjointe de peroxyde d'hydrogène et d'isobutène selon l'une des revendications 1 à 3, caractérisé en ce que la résine échangeuse d'ions de type gel à acidité forte présente une matrice constituée par des chaînes polystyréniques pontées avec du divinylbenzène et dont les groupements acides sont des groupements sulfoniques - $SO_3H$.

5. Procédé de fabrication conjointe de peroxyde et d'isobutène selon la revendication 4, caractérisé en ce que la résine présente, dans le domaine des rayons de pores inférieurs à 0,1 $\mu$m, un volume poreux inférieur à 0,1 cm3/g et, dans le domaine des rayons de pores supérieurs à 0,1 $\mu$m, un volume poreux supérieur à 0,3 cm3/g.

**Claims**

1. Process for jointly producing hydrogen peroxide and isobutene from tertiary butyl hydroperoxide, in which process tertiary butyl hydroperoxide is subjected in an aqueous medium at a temperature of between 60°C and 150°C to the action of an ion exchanger resin in acid form, **characterised in that** the ion exchanger resin is a resin of the gel type having strong, stable acidity in the aqueous medium.

2. Process for jointly manufacturing hydrogen peroxide and isobutene according to claim 1, **characterised in that** the temperature of the aqueous medium is between 80°C and 120°C.

3. Process for jointly manufacturing hydrogen peroxide and isobutene according to one of claims 1 and 2, **characterised in that** the quantity of ion exchanger resin of the gel type having strong acidity which is employed, corresponds at least to 0.005 equivalent $H^+$ per mole of tertiary butyl hydroperoxide used.

4. Process for jointly manufacturing hydrogen peroxide and isobutene according to one of claims 1 to 3, **characterised in that** the ion exchanger resin of the gel type having strong acidity has a matrix constituted by polystyrene chains bridged with divinylbenzene and the acid groups of which are sulfonic groups - $SO_3H$.

5. Process for jointly manufacturing hydrogen peroxide and isobutene according to claim 4, **characterised in that** the resin has, in the range of pore radii of less than 0.1 $\mu$m, a pore volume of less than 0.1 cm3/g and, in the range of pore radii of more than 0.1 $\mu$m, a pore volume greater than 0.3 cm3/g.

**Patentansprüche**

1. Verfahren zur gemeinsamen Herstellung von Wasserstoffperoxid und Isobuten aus Tertiärbutylhydroperoxid, bei dem man das Tertiärbutylhydroperoxid in wäßrigem Medium bei einer Temperatur zwischen 60 °C und 150 °C der Wirkung eines Ionenaustauscherharzes in saurer Form unterzieht, **dadurch gekennzeichnet,** daß das Ionenaustauscherharz ein Harz vom Geltyp mit in dem wäßrigen Medium beständiger starker Azidität ist.

2. Verfahren zur gemeinsamen Herstellung von Wasserstoffperoxid und Isobuten nach Anspruch 1, **dadurch gekennzeichnet,** daß die Temperatur des wäßrigen Mediums zwischen 80 und 120 °C liegt

3. Verfahren zur gemeinsamen Herstellung von Wasserstoffperoxid und Isobuten nach einem der Ansprüche 1 und 2, **dadurch gekennzeichnet,** daß die Menge des besagten Ionenaustauscherharzes vom Geltyp mit starker Azidität wenigstens 0,005 $H^+$- Äquivalenten je Mol des verwendeten Tertiärbutylhydroperoxids entspricht.

4. Verfahren zur gemeinsamen Herstellung von Wasserstoffperoxid und Isobuten nach einem der Ansprüche 1 bis 3, **dadurch gekennzeichnet,** daß das Ionenaustauscherharz vom Geltyp mit starker Azidität eine Matrix bildet, die aus mit Divinylbenzol vernetzten Polystyrolketten besteht und deren saure Gruppen Sulfonsäuregruppen -$SO_3H$ sind.

5. Verfahren zur gemeinsamen Herstellung von Peroxid und Isobuten nach Anspruch 4, **dadurch gekennzeichnet,** daß das Harz im Bereich der Porenradien unterhalb 0,1 $\mu$m ein Porenvolumen unterhalb 0,1 $cm^3$/g und im Bereich der Porenradien oberhalb 0,1 $\mu$m ein Porenvolumen oberhalb 0,3 $cm^3$/g aufweist.